(19) Europäisches Patentamt
European Patent Office
Office européen des brevets

(11) Veröffentlichungsnummer : **0 410 358 B1**

# (12) EUROPÄISCHE PATENTSCHRIFT

(45) Veröffentlichungstag der Patentschrift : **29.09.93 Patentblatt 93/39**

(51) Int. Cl.$^5$ : **C07C 233/65,** C07C 233/66, C07C 233/75, C07C 235/56, C07C 231/02, C07D 295/088, A61K 31/165, A61K 31/535

(21) Anmeldenummer : **90114101.0**

(22) Anmeldetag : **23.07.90**

## (54) Aromatische Carbonsäureamide, ihre Herstellung und Verwendung für Heilmittel.

(30) Priorität : **28.07.89 CH 2818/89**

(43) Veröffentlichungstag der Anmeldung : **30.01.91 Patentblatt 91/05**

(45) Bekanntmachung des Hinweises auf die Patenterteilung : **29.09.93 Patentblatt 93/39**

(84) Benannte Vertragsstaaten : **AT BE CH DE DK ES FR GB GR IT LI LU NL SE**

(56) Entgegenhaltungen :
**EP-A- 0 086 111**
**EP-A- 0 232 199**
**DE-A- 3 202 100**

(73) Patentinhaber : **F. HOFFMANN-LA ROCHE AG**
**Postfach 3255**
**CH-4002 Basel (CH)**

(72) Erfinder : **Klaus, Michael, Dr.**
**Am Hellenrain 6**
**D-7858 Weil/Rhein (DE)**
Erfinder : **Mohr, Peter, Dr.**
**Martinsgasse 9**
**CH-4051 Basel (CH)**

(74) Vertreter : **Lederer, Franz, Dr. et al**
**Lederer, Keller & Riederer, Patentanwälte,**
**Lucile-Grahn-Strasse 22**
**D-81675 München (DE)**

EP 0 410 358 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue aromatische Carbonsäureamide der allgemeinen Formel

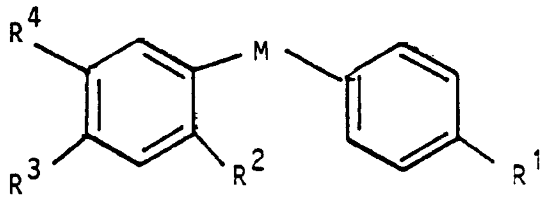

I

worin $R^1$, Halogen oder $OR^5$; $R^2$ Wasserstoff, nieder-Alkyl, nieder-Alkoxy oder Halogen; $R^3$ und $R^4$ unabhängig voneinander nieder-Alkyl; oder zusammengenommen Alkylen mit 3-5 C-Atomen in gerader Kette darstellen; $R^5$ Wasserstoff, Acyl, nieder-Alkoxycarbonyl oder nieder-Alkyl, das durch Amino, mono- oder di-Alkylamino, oder einen Rest -N-Het substituiert sein kann; -N-Het einen über ein N-Atom gebundenen, 5-8-gliedrigen, gesättigten oder ungesättigten monocyclischen Heterocyclus; und M -CONH- oder -NHCO- bedeuten.

Die Erfindung betrifft weiterhin ein Verfahren zur Herstellung der Verbindungen der Formel I, pharmazeutische Präparate auf Basis der Verbindungen der Formel I, die Verbindungen der Formel I als Heilmittel, insbesondere bei der Behandlung und Prophylaxe von Neoplasien, Dermatosen und Altershaut, rheumatischen und immunologischen Erkrankungen sowie die Verwendung der Verbindungen der Formel I bei der Herstellung von pharmazeutischen Präparaten zur Behandlung und Prophylaxe solcher Erkrankungen.

Die Bezeichnung "nieder" bezieht sich auf Gruppen mit 1-6 C-Atomen. Alkyl- und Alkoxygruppen können geradkettig oder verzweigt sein, wie Methyl, Aethyl, Propyl, Isopropyl, Butyl, sek.-Butyl oder tert.-Butyl bzw. Methoxy, Aethoxy, Propoxy, Isopropoxy, Butoxy, sek.-Butoxy und tert.-Butoxy. Beispiele von Acylgruppen sind Alkanoylgruppen, vorzugsweise nieder-Alkanoylgruppen, wie Acetyl, Propionyl, Butyryl, Pivaloyl und Caproyl; oder Aroylgruppen wie Benzoyl, p-Nitrobenzoyl und Toluoyl; oder Aralkanoylgruppen wie Phenylacetyl.

Bevorzugte heterocyclische Reste -NHet sind solche der Formel -NY, wobei Y $-CH_2-$, -CH=, -O-, -S-, -SO-, $-SO_2-$ oder $-NR^6$ - und $R^6$ Wasserstoff, nieder-Alkyl oder Acyl ist und wobei zwischen N und Y insgesamt 3-6 C-Atome angeordnet sind. Beispiele solcher Reste sind Piperidino, Pyrrolidino, Morpholino, Piperazino, N-Methylpiperazino, Thiomorpholino, Thiomorpholino-4-oxid, Thiomorpholino-4,4-dioxid sowie Imidazolino und Pyrrolo. Ein durch $R^3$ und $R^4$ gemeinsam dargestellter Alkylenrest mit 3-5 C-Atomen in gerader Kette kann Verzweigungen aufweisen, Beispiele für solche Alkylenreste sind 1,3-Propylen, 1,4-Butylen und 1,5-Pentylen und nieder-Alkyl-substituierte Derivate davon, wie die Reste $-C(CH_3)_2-CH_2-C(CH_3)_2-$, $-C(CH_3)_2-CH_2CH_2-C(CH_3)_2-$ und $-CH_2CH_2-C(CH_3)_2-CH_2CH_2-$.

Bevorzugte Reste $R^1$ sind Hydroxy, Fluor, Morpholinoäthoxy und N-Methylpiperidinoäthoxy.

Das Dokument DE-A1-32 02 100 beschreibt Alkyl-substituierte Tetrahydronaphthyl- und Indanylvinylbenzoesäure-4-hydroxy-anilide, die zur Behandlung von Neoplasien, Akne, Psoriasis, dermatologischen Affektionen und rheumatischen Erkrankungen geeignet sein sollen. Das Dokument EP-A 0 232 199 beschreibt substituierte Benzoesäureamide, die bei der Behandlung von mit Keratinisierungsstörungen verbundenen dermatologischen Affektio nen und solchen mit entzündlicher und immunallergischer Komponente, degenerativen Erkrankungen des Bindegewebes und rheumatoider Psoriasis wie auch in Kosmetika verwendet werden sollen.

Die Verbindungen der Formel I können dadurch erhalten werden, dass man

a) eine Verbindung der allgemeinen Formel

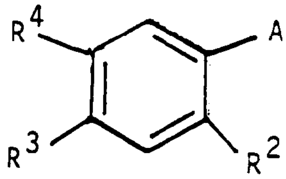

II

;

mit einer Verbindung der allgemeinen Formel

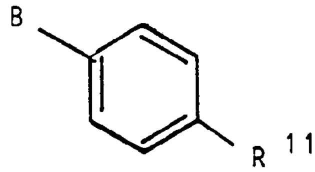

III

umsetzt,

wobei entweder A eine Carboxylgruppe oder ein reaktionsfähiges Derivat davon und B eine Aminogruppe bedeutet, oder A eine Aminogruppe und B eine Carboxylgruppe oder ein reaktionsfähiges Derivat davon und $R^{11}$ einen Rest $R^1$ bedeutet, in dem eine gegebenenfalls anwesende Aminogruppe in geschützter Form vorliegt,

worauf man eine gegebenenfalls anwesende Aminoschutzgruppe abspaltet.

Die Umsetzung einer Verbindung II mit einer Verbindung III kann nach an sich bekannten Methoden für die Acylierung von Aminen durchgeführt werden. Vorzugsweise wird eine Verbindung der Formel II, in der A eine Carbonsäurehalogenidgruppe, z.B. die Gruppe -COCl darstellt, mit einer Verbindung der Formel III, in der B $-NH_2$ ist, zu einer Verbindung der Formel I, in der M -CONH- ist, umgesetzt, oder ein Amin der Formel II mit einem Carbonsäurehalogenid der Formel III zu einer Verbindung der Formel I umgesetzt, in der M -NH-CO- ist.

Diese Acylierungen werden zweckmässig in Gegenwart einer Base, z.B. einer organischen Base wie Pyridin, durchgeführt.

Als Aminoschutzgruppen kommen konventionelle Aminoschutzgruppen in Betracht wie die Phthaloylgruppe, die Benzyloxycarbonylgruppe und die tert.-Butoxycarbonylgruppe. Die Abspaltung dieser Schutzgruppen kann mit konventionellen Mitteln erfolgen. Eine Phthaloylgruppe kann durch Behandlung mit Hydrazin; eine Benzyloxycarbonylgruppe kann durch katalytische Hydrierung; und eine tert.-Butoxycarbonylgruppe kann durch Behandlung mit Säuren, z.B. verdünnter Salzsäure oder Trifluoressigsäure, abgespalten werden.

Die als Ausgangsmaterialien für die Herstellung der Verbindungen der Formel I verwendeten Verbindungen der Formeln II und III können, soweit sie nicht bekannt oder nachstehend beschrieben sind, in Analogie zu bekannten oder den nachstehend beschriebenen Methoden hergestellt werden.

Die Verfahrensprodukte der Formel I stellen pharmakodynamisch wertvolle Verbindungen dar. Sie können zur topischen und systemischen Therapie von benignen und malignen Neoplasien, von prämalignen Läsionen, sowie ferner auch zur systemischen und topischen Prophylaxe der genannten Affektionen verwendet werden.

Sie sind des weiteren für die topische und systemische Therapie von Akne, Psoriasis und anderen mit einer verstärkten oder pathologisch veränderten Verhornung einhergehenden Dermatosen, wie auch von entzündlichen und allergischen dermatologischen Affektionen sowie lichtgeschädigter (Alters)haut geeignet. Die Verfahrensprodukte der Formel I können ferner auch zur Bekämpfung von Schleimhauterkrankungen mit entzündlichen oder degenerativen bzw. metaplastischen Veränderungen eingesetzt werden. Die antineoplastische Wirksamkeit der Verbindungen der Formel I kann mit der nachstehend beschriebenen Versuchsanordnung geprüft werden.

Weibliche Sprague-Dawley-Ratten werden unter Temperatur- und Licht-kontrollierten Bedingungen gehalten, bei freiem Zugang zu Trinkwasser und Futter. Im Alter von 50 Tagen werden jeder Ratte 12 mg 7,12-Dimethylbenz(a)anthracen mittels einer Magensonde verabreicht. Nach einer Zeit von ca. 4 Monaten, in der sich durchschnittlich 3,6 bis 4 Mammatumoren pro Ratte entwickelt haben, wird mit der Behandlung begonnen. Die Versuchssubstanz wird in einer 25%igen sprühgetrockneten Formulierung zum normalen Futter zugemischt. Die folgenden Parameter werden wöchentlich gemessen: Körpergewicht, mittlere Tumorzahl und mittleres Tumorvolumen pro Tier. Die Volumen werden nach der Formel $\frac{D}{2} \bullet d^2$ berechnet, wobei D der grössere und d der kleinere Durchmesser des Tumorellipsoids darstellt.

Die mit der Verbindung von Beispiel 3 in dieser Versuchsanordnung nach einer Versuchsdauer von 10 Wochen erhaltenen Werte sind in Tabelle I dargestellt:

Tabelle I

| Dosis [mg/kg/Tag] p.o. | Veränderung der durchschnittlichen Anzahl von Tumoren pro Ratte [%] | Aenderung des durchschnittlichen Tumorvolumens pro Ratte [%] |
|---|---|---|
| 50 | +50 | +840 |
| 100 | +35 | +475 |
| 200 | +16 | +193 |
| Kontrollgruppe | +78 | +875 |

Die Verbindungen der Formel I können auch zur Behandlung entzündlicher, allergischer, rheumatischer und immunologischer Erkrankungen der verschiedensten Organe verwendet werden. Beispiele solcher Krankheiten sind: Primär-chronische Polyarthritis, Spondylarthritis ancylopoetica, Osteoarthritiden, Arthritiden und Arthrosen; Ekzeme, atopische Dermatitis, Rhinitis allergica, Asthma bronchiale; Autoimmunkrankheiten wie z.B. Lupus erythematosus, Reiter's Syndrom.

Die Verbindungen der Formel I können deshalb als Heilmittel, z.B. in Form pharmazeutischer Präparate, Anwendung finden.

Die Mittel können enteral, parenteral oder topisch verabreicht werden. Für die enterale Applikation eignen sich z.B. Mittel in Form von Tabletten, Kapseln, Dragees, Sirupen, Suspensionen, Lösungen und Suppositorien. Für die parenterale Applikation sind Mittel in Form von Infusions- oder Injektionslösungen geeignet.

Die Dosierungen, in denen die Präparate verabreicht werden, können je nach Anwendungsart und Anwendungsweg sowie nach den Bedürfnissen der Patienten variieren.

Bei oraler Verabreichung der erfindungsgemässen Verbindungen kommen beim Erwachsenen Dosierungen von etwa 0,1-100 mg/kg, vorzugsweise 0,5-50 mg/kg, pro Tag in Betracht.

Die Präparate können in einer oder mehreren Dosierungen verabreicht werden. Eine bevorzugte Darreichungsform sind Kapseln mit einem Gehalt von ca. 5-500 mg Wirkstoff.

Die Präparate können inerte oder auch pharmakodynamisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Zur topischen Anwendung werden die Wirkstoffe zweckmässig in Form von Salben, Tinkturen, Crèmen, Lösungen, Lotionen, Sprays, Suspensionen und dergleichen verwendet. Bevorzugt sind Salben und Crèmen sowie Lösungen. Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,1-5%ige, vorzugsweise 0,3-2%ige Lösungen, sowie ca. 0,1-5%ige, vorzugsweise ca. 0,3-2%ige Salben oder Crèmen geeignet.

Den Präparaten kann gegebenenfalls ein Antioxydationsmittel, z.B. Tocopherol, N-Methyl-γ-tocopheramin

sowie t-Butyl-Hydroxyanisol oder t-Butyl-Hydroxytoluol beigemischt sein.

Die Verbindungen der Formel I können, wie in dem nachstehenden Referenzbeispiel, das nicht Gegenstand der Erfindung ist, beschrieben hergestellt werden.

Referenzbeispiel

2 g 5,6,7,8-Tetrahydro-5,5,8,8-tetramethyl-2-naphthalincarbonsäure wurden mit 15 ml Thionylchlorid versetzt und 1 Stunde am Rückfluss erwärmt. Nach dem Abdestillieren des überschüssigen Thionylchlorides wurde der Rückstand in 10 ml Tetrahdrofuran gelöst und unter Rühren zu einer Lösung von 0,9 g Anilin in 20 ml Pyridin hinzugetropft. Nach halbstündigem Rühren bei Raumtemperatur wurde das Reaktionsgemisch auf Eiswasser gegossen, mit Essigester extrahiert, die organische Phase mit 2N Salzsäure und Wasser gewaschen, getrocknet und eingedampft. Das orangefarbene Oel wurde aus Hexan kristallisiert und ergab 1,8 g 5,6,7,8-Tetrahydro-5,5,8,8-tetramethyl -2-naphthalin-carboxanilid in farblosen Kristallen, Smp. 144-146°C.

Beispiel 1

In Analogie zum Referenzbeispiel wurden aus 2 g 5, 6, 7, 8-Tetrahydro-5, 5, 8, 8-tetramethyl-2-naphthalincarbonsäure und 1 g 4-Fluoranilin 1,7 g 4'-Fluor-5, 6, 7, 8-tetrahydro-5, 5, 8, 8-tetramethyl-2-naphthalin-carboxanilid in farblosen Kristallen hergestellt, Smp. 163-165 ° C (aus Essig-ester/Hexan).

Beispiel 2

In Analogie zum Referenzbeispiel wurden 52,5 g 5,6,7,8-Tetrahydro-5,5,8,8-tetramethyl-2-naphthalincarbonsäure durch Reaktion mit 200 ml Thionylchlorid in das Säurechlorid überführt und nach Lösen in 200 ml Tetrahydrofuran zu einer Lösung von 48,8 g 4-[2-(4-Amino-phenoxy)äthyl]morpholin in 400 ml Pyridin unter leichter Kühlung zugetropft. Die Temperatur sollte nicht über 30°C steigen. Nach 1-stündigem Rühren bei Raumtemperatur wurde das Reaktionsgemisch auf Eiswasser gegossen, mit Essigester extrahiert, die organische Phase mit eiskalter 2N Salzsäure gewaschen, getrocknet und eingedampft. Das kristalline Rohprodukt wurde durch Filtration über eine Kieselgelsäule (Eluierungsmittel Hexan/Essigester 1:4, dann Essigester, dann Essigester/Aethanol = 1:1) gereinigt und aus Hexan/Essigester umkristallisiert. Man erhielt 52 g 5,6,7,8-Tetrahydro-5,5,8,8-tetramethyl -4'-(2-morpholinoäthoxy)-2-naphthalin-carboxanilid in weissen Kristallen, Smp. 134-136°C.

Das als Ausgangsmaterial verwendete 4-[2-(4-Amino-phenoxy)äthyl]morpholin wurde wie folgt hergestellt:

19 g Natriumhydrid (50%ige Suspension in Mineralöl) wurden mit absolutem Pentan zweimal gewaschen, getrocknet und in 130 ml Dimethylformamid suspendiert. Unter Eiskühlung wurde eine Lösung von 42,5 g 4-Aminophenol in 250 ml Dimethylformamid hinzugetropft und eine weitere Stunde bei 0°C gerührt. Danach wurde eine Lösung von 100 g 4-(2-Chloräthyl)-morpholin in 250 ml Dimethylformamid tropfenweise zugegeben. Nach 1-stündigem Erwärmen auf 70°C wurde das Reaktionsgemisch auf Eiswasser gegossen, mit Essigester extrahiert, die organische Phase mit Wasser gewaschen, getrocknet und eingedampft. Das so erhaltene dunkelbraune Oel wurde durch Filtration über eine Kieselgelsäule (Eluierungsmittel Essigester) gereinigt und ergab nach dem Trocknen im Hochvakuum 66 g 4-[2-(4-Aminophenoxy)äthyl]morpholin als leicht bräunliches Oel.

Beispiel 3

In Analogie zum Referenzbeispiel erhielt man aus 2 g 5,6,7,8-Tetrahydro-5,5,8,8-tetramethyl-2-naphthalincarbonsäure und 0,95 g 4-Aminophenol nach Umkristallisation aus Hexan/Essigester 2,1 g 5,6,7,8-Tetrahydro-5,5,8,8-tetramethyl -4'-hydroxy-2-naphthalincarboxanilid in beigen Kristallen, Smp. 216-218°C.

Beispiel 4

In Analogie zum Referenzbeispiel erhielt man aus 4 g 1,1,3,3-Tetramethyl-5-indancarbonsäure und 2 g 4-Fluoranilin nach Umkristallisation aus Hexan/Essigester 2,1 g 4'-Fluor-1,1,3,3-tetramethyl-5-indancarboxanilid, Smp. 155-156°C.

Beispiel 5

In Analogie zu Beispiel 2 ergab die Umsetzung von 5,7 g 1,1,3,3-Tetramethyl-5-indancarbonsäure mit 5,8

g 4-[2-(4-Aminophenoxy)äthyl]morpholin nach Umkristallisation aus Hexan/Essigester 5,2 g 1,1,3,3-Tetramethyl-4'-(2-morpholinoäthoxy) -5-indancarboxanilid in weissen Kristallen, Smp. 131-133°C.

Beispiel 6

In Analogie zum Referenzbeispiel erhielt man aus 4 g 1,1,3,3-Tetramethyl-5-indancarbonsäure und 1,9 g 4-Aminophenol nach Umkristallisation aus Hexan/Essigester 2,3 g 4'-Hydroxy-1,1,3,3-tetramethyl-5-indancarboxanilid, Smp. 196-197°C.

Beispiel 7

Durch Umsetzung von 5,9 g 5,6,7,8-Tetrahydro-5,5,8,8-tetramethyl-2-naphthylamin mit dem aus 4 g 4-Fluorbenzoesäure und 15 ml Thionylchlorid gewonnenen 4-Fluorbenzoylchlorid wurde nach Umkristallisation aus Hexan/Essigester 6,3 g p-Fluor-N-(5,6,7,8-tetrahydro-5,5,8,8-tetramethyl -2-naphthyl)benzamid hergestellt, Smp. 160-162°C.

Beispiel 8

6,1 g p-(2-Morpholinoäthoxy)benzoesäure werden mit 100 ml Thionylchlorid übergossen und 1 Stunde am Rückfluss erwärmt. Nach Abdampfen des überschüssigen Thionylchlorides wurde der Rückstand in 100 ml Tetrahydrofuran suspendiert und zu einer Lösung von 4,9 g 5,6,7,8-Tetrahydro-5,5,8,8-tetramethyl-2-naphthylamin in 150 ml Pyridin getropft. Nach 20-stündigem Rühren bei Raumtemperatur wurde das Reaktionsgemisch auf Eiswasser gegossen und mit Essigester extrahiert. Nach mehrmaligem Waschen der organischen Phase mit Wasser, Trocknen über Natriumsulfat und Abdampfen des Lösungsmittels erhielt man ein kristallines Rohprodukt, das durch Filtration über eine Kieselgel-Säule (Eluierungsmittel Hexan/Essigester = 1:1, dann Essigester) und Kristallisation aus Hexan/Essigester weiter gereinigt wurde. Man erhielt 8,6 g p-(2-Morpholinoäthoxy)-N-(5,6,7,8-tetrahydro -5,5,8,8-tetramethyl-2-naphthyl)benzamid, Smp. 130-132°C.

Die als Ausgangsprodukt verwendete p-(2-Morpholinoäthoxy)benzoesäure wurde wie folgt hergestellt:

10 g 4-Hydroxy-benzoesäuremethylester und 20,5 g 4-(2-Chloräthylmorpholin wurden in 100 ml Dimethylformamid gelöst und nach Zugabe von 38 g Kaliumcarbonat während 1 Stunde auf 100°C erwärmt. Das erhaltene Reaktionsgemisch wurde auf Eiswasser gegossen, mit Essigester extrahiert, getrocknet und eingedampft. Der ölige, schwach braune Rückstand wurde mit Kaliumhydroxid in Wasser/Aethanol verseift und ergab nach Ansäuern und Umkristallisation aus Hexan/Essigester 7,3 g p-(2-Morpholinoäthoxy)benzoesäure in beigen Kristallen, Smp. 112-114°C.

Beispiel 9

Aus 1 g 5,6,7,8-Tetrahydro-5,5,8,8-tetramethyl-2-naphthylamin und 1 g p-Acetoxybenzoylchlorid wurde nach Umkristallisation aus Hexan/Essigester 1,5 g p-[(5,6,7,8-Tetrahydro-5,5,8,8-tetramethyl -2-naphthyl)carbamoyl]phenylacetat, Smp. 186-188°C erhalten.

Hydrolyse dieser Verbindung mit Kaliumhydroxid/Wasser/Aethanol ergab nach Umkristallisation aus Hexan/Essigester 1,1 g p-Hydroxy-N-(5,6,7,8-tetrahydro-5,5,8,8-tetramethyl -2-naphthyl)benzamid. Smp. 204-206°C.

Beispiel 10

Aus 5,5 g 1,1,3,3-Tetramethyl-5-indanamin und 4 g 4-Fluorbenzoylchlorid wurde nach Umkristallisation aus Hexan/Essigester 5,5 g p-Fluor-N-(1,1,3,3-tetramethyl -5-indanyl)benzamid, Smp. 167-169°C. erhalten.

Beispiel 11

In Analogie zu Beispiel 8 erhielt man aus 4 g p-(2-Morpholinoäthoxy)benzoesäure und 3,1 g 1,1,3,3-Tetramethyl-5-indanamin nach Umkristallisation aus Hexan/Essigester 4,6 g p-(2-Morpholinoäthoxy)-N-(1,1,3,3-tetramethyl -5-indanyl)benzamid, Smp. 134-136°C.

Beispiel 12

In Analogie zu Beispiel 9 erheilt man aus 2 g 1,1,3,3-Tetramethyl-5-indanamin und 2,2 g p-Acetoxybenzoylchlorid nach Umkristallisation aus Hexan/Essigester 2,7 g p-[1,1,3,3-Tetramethyl-5-indanoyl]carbamoyl]phenylacetat, Smp. 196-198°C.

Hydrolyse dieser Verbindung ergab 2,2 g p-Hydroxy-N-(1,1,3,3-tetramethyl -5-indanyl)benzamid, Smp. 185-187°C.

Beispiel 13

1,77 g 6,7,8,9-Tetrahydro-7,7-dimethyl-5H-benzocyclohepten -2-carbonsäure wurden mit 1,17 ml $SOCl_2$ versetzt und während 3/4 Stunden zum Rückfluss erhitzt. Das überschüssige Reagens wird unter vermindertem Druck entfernt und das rohe Säurechlorid kurz am Hochvakuum getrocknet. Dann löst man es in 20 ml abs. Pyridin und tropft es unter Argonatmosphäre bei 0°C zu einer Lösung von 0,97 g 4-Aminophenol in 16 ml abs. Pyridin. Man lässt 20 Minuten bei Raumtemperatur nachreagieren und giesst dann auf Eis/konz. HCl. Dann wird mit Aethylacetat extrahiert, mit 1N HCl, 10% Soda- und gesättigter NaCl-Lösung gewaschen, über $Na_2SO_4$ getrocknet, mit Aktivkohle aufgekocht und filtriert. Man engt das Filtrat unter vermindertem Druck ein, bis das Produkt zu kristallisieren beginnt und erhält 1,98 g 6,7,8,9-Tetrahydro-7,7-dimethyl-5H-benzocyclohepten-2-carbonsäure-(4-hydroxy)anilid als bräunliche Kristalle vom Schmelzpunkt 175-176°C.

Das Edukt kann wie folgt hergestellt werden:

Zu 16,3 g $Ca(OCl)_2$ in 60 ml $H_2O$ gibt man eine Mischung von 11,4 g $K_2CO$ und 3,40 g KOH, löst in 30 ml $H_2O$. Man rührt 1/4 Stunde intensiv und filtriert dann ab. Zum Filtrat (Kalciumhypochlorit) gibt man 6,49 g 6,7,8,9-Tetrahydro-7,7-dimethyl-5H-benzocyclohepten-2-yl-methylketon und erhitzt langsam. Bei 70°C setzt eine stark exotherme Reaktion ein, welche die Temperatur auf 100°C ansteigen lässt. Nach dem Abkühlen säuert man vorsichtig mit 50 ml 3N HCl an und filtriert die ausgefallene Säure ab. Nach Waschen mit $H_2O$ und Trocknen am Hochvakuum erhält man 5,48 g 6,7,8, 9-Tetrahydro-7,7-dimethyl-5H-benzocyclohepten -2-carbonsäure als weisse Kristalle vom Schmelzpunkt 166-171°C.

Beispiel 14

In Analogie zu Beispiel 13, aber mit 4-Fuoranilin als Amin-Komponente, wurde die 6,7,8,9-Tetrahydro-7,7-dimethyl-5H-benzocyclohepten -2-carbonsäure(4-fluor)anilid als weisse Kristalle vom Schmelzpunkt 178-179°C hergestellt.

Beispiel 15

In Analogie zu Beispiel 13 wurde aus 4-Aminophenol und 6,7,8,9-Tetrahydro-9,9-dimethyl-5H-benzocyclohepten-2-carbonsäure das 6,7,8,9-Tetrahydro-9,9-dimethyl-5H-benzocyclohepten-2-carbonsäure(4-hydroxy)anilid als weisse Kristalle vom Schmelzpunkt 147-148°C hergestellt.

Die Ausgangsverbindung kann wie folgt hergestellt werden:

Das 4,00 g 2-Brom-6,7,8,9-tetrahydro-9,9-dimethyl-5H-benzocyclohepten (EP-A2-0315071) und 456 mg Mg-Spänen wurde unter Argon-Atmosphäre in 20 ml absolutem Tetrahydrofuran die Grignard-Verbindung hergestellt. Nach erfolgter Metallierung wird bei -10°C ein kräftiger $CO_2$-Strom eingeleitet. Man hydrolysiert mit verd. HCl, extrahiert mit Aether und wäscht mit wenig Wasser. Dann reinigt man die Säure durch Extraktion in 1N NaOH, Ansäuern auf pH 1 (HCl) und Rückextraktion in Aether. Nach Waschen mit Wasser, Trocknen über $Na_2SO_4$ und Eindampfen erhält man 2,64 g 6,7,8,9-Tetrahydro-9,9-dimethyl-5H-benzocyclohepten -2-carbonsäure als farblose Kristalle vom Schmelzpunkt 155-156°C.

Beispiel 16

In Analogie zu Beispiel 13 wurde aus 4-Fluoranilin und 6,7,8,9-Tetrahydro-9,9-dimethyl-5H-benzocyclohepten -2-carbonsäure das 6,7,8,9-Tetrahydro-9,9-dimethyl-5H-benzocyclohepten-2-carbonsäure(4-fluor)anilid als farblose Kristalle vom Schmelzpunkt 135-136°C hergestellt.

Beispiel 17

In Analogie zu Beispiel 13, wurde ausgehend von 3-Aethyl-6,7,8,9-tetrahydro-7,7-dimethyl-5H-benzocyclohepten -2-yl-methylketon und mit 4-Aminophenol als Aminkomponente, das 3-Aethyl-6,7,8,9-tetrahydro-7,7-dimethyl-5H-benzocyclohepten -2-carbonsäure(4-hydroxy)anilid als farblose Kristalle vom Schmelzpunkt 197-198°C hergestellt.

Das Ausgangsmaterial kann aus den Bausteinen Aethylbenzol, 3.3-Dimethylglutarsäureanhydrid und Acetylchlorid synthetisiert werden (vgl. EP-A2-0315071).

Beispiel 18

Weitere Beispiele erfindungsgemäss erhältlicher Verbindungen sind

6,7,8,9-Tetrahydro-7,7-dimethyl-4'-[2-(4-methylpiperazino)äthoxy]-(5H)-2-benzocyclohepten-carboxanilid;

6,7,8, 9-Tetrahydro-4'-hydroxy-5,5-dimethyl-5H-benzocyclohepten-2-carbonsäureanilid;

4'-[2-[Bis(2-methoxyäthyl)amino]äthoxy]-6,7,8,9-tetrahydro-6,6,8,8-tetramethyl-5H-benzocyclohepten- 2-carbonsäureanilid;

5,6,7,8-Tetrahydro-5,5,8,8-tetramethyl-4'-[2-(4-methylpiperazino)äthoxy]-2-naphthalin-carboxanilid;

1,1,3,3-Tetramethyl-4'-(2-dimethylaminoäthoxy)-5-indancarboxanilid.

p-Fluor-N-(6,7,8,9-tetrahydro-7,9,9-trimethyl-5H-benzocyclohepten-2-yl)benzamid;

p-(2-Imidazoloäthoxy)-N-(5,6,7,8-tetrahydro-5,5,8,8-tetramethyl-2-naphthyl)benzamid;

p-(2-Morpholinoäthoxy)-N-(6,7,8,9-tetrahydro-7,7-dimethyl-5H-benzocyclohepten-2-yl)benzamid; und

6,7,8,9-Tetrahydro-7,7-dimethyl-4'-[2-(4-methylpiperazino)äthoxy]-(5H)-2-benzocyclohepten-carboxanilid.

Beispiel A

Hartgelatinekapseln können wie folgt hergestellt werden:

| | Bestandteile | | mg/Kapsel |
|---|---|---|---|
| 1. | sprühgetrocknetes Pulver enthaltend 75% Verbindung I | | 200 |
| 2. | Natriumdioctylsulfosuccinat | | 0,2 |
| 3. | Natriumcarboxymethylcellulose | | 4,8 |
| 4. | mikrokristalline Cellulose | | 86,0 |
| 5. | Talk | | 8,0 |
| 6. | Magnesiumstearat | | 1,0 |
| | | Total | 300 |

Das sprühgetrocknete Pulver, das auf dem Wirkstoff, Gelatine und mikrokristalliner Cellulose basiert und eine mittlere Korngrösse des Wirkstoffes von <1 μ aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

Beispiel B

Tabletten können wie folgt hergestellt werden:

| Bestandteile | | mg/Tablette |
|---|---|---|
| 1. | Verbindung I als feingemahlenes Pulver | 500 |
| 2. | Milchzucker pulv. | 100 |
| 3. | Maisstärke weiss | 60 |
| 4. | Povidone K30 | 8 |
| 5. | Maisstärke weiss | 112 |
| 6. | Talk | 16 |
| 7. | Magnesiumstearat | 4 |
| | Total | 800 |

Die feingemahlene Substanz wird mit Milchzucker und einem Teil der Maisstärke gemischt. Die Mischung wird mit einer wässrigen Lösung von Povidone K30 befeuchtet und geknetet, und die resultierende Masse granuliert, getrocknet und gesiebt. Das Granulat wird mit der restlichen Maisstärke, Talk und Magnesiumstearat vermischt und zu Tabletten geeigneter Grösse verpresst.

Beispiel C

Weichgelatinekapseln können wie folgt hergestellt werden:

| Bestandteile | | mg/Kapsel |
|---|---|---|
| 1. | Verbindung I | 50 |
| 2. | Triglycerid | 450 |
| | Total | 500 |

10 g Verbindung I werden unter Rühren, Inertbegasung und Lichtschutz in 90 g mittelkettigem Triglycerid gelöst. Diese Lösung wird als Kapselfüllmasse zu Weichgelatinekapseln a 50 mg Wirkstoff verarbeitet.

Beispiel D

Eine Lotion kann wie folgt hergestellt werden:

| Bestandteile | | |
|---|---|---|
| 1. | Verbindung I, feingemahlen | 3,0 g |
| 2. | Carbopol 934 | 0,6 g |
| 3. | Natriumhydroxid | q.s. ad pH 6 |
| 4. | Aethanol, 94% | 50,0 g |
| 5. | entmineralisiertes Wasser | ad 100,0 g |

Der Wirkstoff wird unter Lichtschutz in die Mischung Aethanol, 94%ig/Wasser eingearbeitet. Carbopol 934 wird bis zur vollständigen Gelierung eingerührt und der pH-Wert mit Natriumhydroxid eingestellt.

**Patentansprüche**

**Patentansprüche für folgende Vertragsstaaten : AT, BE, CH, DE, DK, FR, GB, IT, LI, LU, NL, SE**

1. Verbindungen der allgemeinen Formel

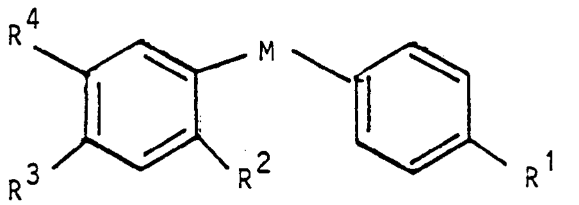

I

worin $R^1$ , Halogen oder $OR^5$; $R^2$ Wasserstoff, nieder-Alkyl, nieder-Alkoxy oder Halogen; $R^3$ und $R^4$ unabhängig voneinander nieder-Alkyl; oder zusammengenommen Alkylen mit 3-5 C-Atomen in gerader Kette darstellen; $R^5$ Wasserstoff, Acyl, nieder-Alkoxycarbonyl oder nieder-Alkyl, das durch Amino, mono- oder di-Alkylamino, oder einen Rest -N-Het substituiert sein kann; -N-Het einen über ein N-Atom gebundenen, 5-8-gliedrigen, gesättigten oder ungesättigten monocyclischen Heterocyclus; und M -CONH- oder -NHCO- bedeuten.

2. Verbindungen gemäss Anspruch 1, worin $R^3$ und $R^4$ zusammengenommen Alkylen mit 3-5 C-Atomen in gerader Kette darstellen..

3. Verbindungen gemäss Anspruch 2, worin $R^3$ und $R^4$ zusammengenommen die Reste $-C(CH_3)_2-CH_2-C(CH_3)_2-$, $-C(CH_3)_2-CH_2CH_2-C(CH_3)_2-$ und $-CH_2CH_2-$ $-C(CH_3)_2-CH_2CH_2-$ sind.

4. Verbindungen gemäss den Ansprüchen 1-3, worin $R^1$ Hydroxy, Fluor, Morpholinoäthoxy oder N-Methylpiperidinoäthoxy ist.

5. Verbindungen gemäss den Ansprüchen 1-4, in denen M -CONH- ist.

6. 5,6,7,8-Tetrahydro-5,5,8,8-tetramethyl-4'-(2-morpholinoäthoxy)-2-naphthalin-carboxanilid.

7. 5,6,7,8-Tetrahydro-5,5,8,8-tetramethyl-4'-hydroxy-2-naphthalincarboxanilid.

8. Die Verbindungen 4'-Fluor-5,6,7,8-tetrahydro-5,5,8,8-tetramethyl-2-naphthalin-carboxanilid; 4'-Fluor-1,1,3,3-tetramethyl-5-indancarboxanilid; 1,1,3,3-Tetramethyl-4'-(2-morpholinoäthoxy) -5-indancarboxanilid; 4'-Hydroxy-1,1,3,3-tetramethyl-5-indancarboxanilid; 6,7,8,9-Tetrahydro-7,7-dimethyl-5H-benzocyclohepten-2-carbonsäure(4-hydroxy)anilid; 6,7,8,9-Tetrahydro-7,7-dimethyl-5H-benzocyclohepten -2-carbonsäure(4-fluor)anilid;

9. Verbindungen gemäss den Ansprüchen 1-4, in denen M -NHCO- ist.

10. (5,6,7,8-Tetrahydro-5,5,8,8-tetramethyl-2-naphthyl)-4-hydroxybenzamid.

11. Die Verbindungen p-Fluor-N-(5,6,7,8-tetrahydro-5,5,8,8-tetramethyl -2-naphthyl)benzamid; p-(2-Morpholinomethoxy)-N-(5,6,7,8-tetrahydro-5,5,8,8-tetramethyl-2-naphthyl)benzamid.

12. Die Verbindungen p-Fluor-N-(1,1,3,3-tetramethyl -5-indanyl)benzamid; p-(2-Morpholinoäthoxy)-N-(1,1,3,3-tetramethyl -5-indanyl)benzamid; p-Hydroxy-N-(1,1,3,3-tetramethyl -5-indanyl)benzamid; 6,7,8,9-Tetrahydro-9,9-dimethyl-5H-benzocyclohepten-2-carbonsäure(4-hydroxy)anilid; 6,7,8,9-Tetrahydro-9,9-dimethyl-5H-benzocyclohepten-2-carbonsäure(4-fluor)anilid; 3-Aethyl-6,7,8,9-tetrahydro-7,7-dimethyl-5H-benzocyclohepten -2-carbonsäure(4-hydroxy)anilid.

13. Die Verbindungen der Formel I zur Anwendung als Heilmittel.

14. Verfahren zur Herstellung der Verbindungen von Anspruch 1, dadurch gekennzeichnet, dass man
a) eine Verbindung der allgemeinen Formel

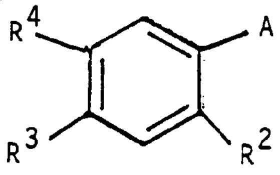

II

mit einer Verbindung der allgemeinen Formel

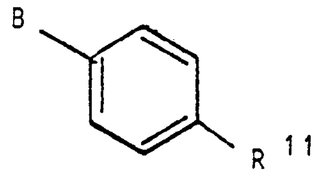

III

umsetzt,

wobei entweder A eine Carboxylgruppe oder ein reaktionsfähiges Derivat davon und B eine Aminogruppe bedeutet, oder A eine Aminogruppe und B eine Carboxylgruppe oder ein reaktionsfähiges Derivat davon und $R^{11}$ einen Rest $R^1$ bedeutet, in dem eine gegebenenfalls anwesende Aminogruppe in geschützter Form vorliegt,

worauf man eine gegebenenfalls anwesende Aminoschutzgruppe abspaltet.

**15.** Pharmazeutische Präparate, enthaltend eine Verbindung der Formel I und übliche Träger- und Hilfsstoffe.

**16.** Verwendung einer Verbindung der Formel I zur Herstellung von pharmazeutischen Präparaten zur Behandlung von Neoplasien und Dermatosen.

**Patentansprüche für folgende Vertragsstaaten : ES, GR**

**1.** Verfahren zur Herstellung von Verbindungen der allgemeinen Formel

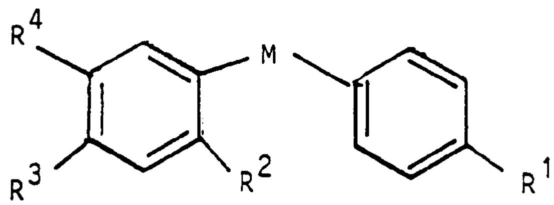

I

worin $R^1$ Halogen oder $OR^5$; $R^2$ Wasserstoff, nieder-Alkyl, nieder-Alkoxy oder Halogen; $R^3$ und $R^4$ unabhängig voneinander nieder-Alkyl; oder zusammengenommen Alkylen mit 3-5 C-Atomen in gerader Kette darstellen; $R^5$ Wasserstoff, Acyl, nieder-Alkoxycarbonyl oder nieder-Alkyl, das durch Amino, mono- oder di-Alkylamino, oder einen Rest -N-Het substituiert sein kann; -N-Het einen über ein N-Atom gebundenen, 5-8-gliedrigen, gesättigten oder ungesättigten monocyclischen Heterocyclus; und M -CONH- oder -NHCO- bedeuten,

dadurch gekennzeichnet, dass man

a) eine Verbindung der allgemeinen Formel

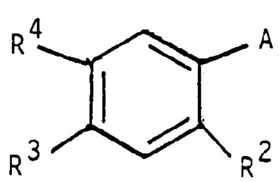

II

mit einer Verbindung der allgemeinen Formel

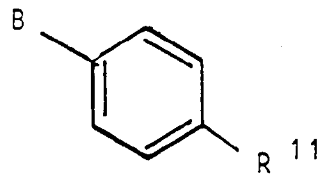

III

umsetzt,

wobei entweder A eine Carboxylgruppe oder ein reaktionsfähiges Derivat davon und B eine Aminogruppe bedeutet, oder A eine Aminogruppe und B eine Carboxylgruppe oder ein reaktionsfähiges Derivat davon und $R^{11}$ einen Rest $R^1$bedeutet, in dem eine gegebenenfalls anwesende Aminogruppe in geschützter Form vorliegt,

worauf man eine gegebenenfalls anwesende Aminoschutzgruppe abspaltet.

**2.** Verfahren gemäss Anspruch 1, wobei $R^3$ und $R^4$ zusammengenommen Alkylen mit 3-5 C-Atomen in gerader Kette darstellen..

**3.** Verfahren gemäss Anspruch 2, wobei $R^3$ und $R^4$ zusammengenommen die Reste $-C(CH_3)_2-CH_2-C(CH_3)_2-$, $-C(CH_3)_2-CH_2CH_2-C(CH_3)_2-$ und $-CH_2CH_2-C(CH_3)_2-CH_2CH_2-$ sind.

**4.** Verfahren gemäss den Ansprüchen 1-3, worin $R^1$ Hydroxy, Fluor, Morpholinoäthoxy oder N-Methylpiperidinoäthoxy ist.

**5.** Verfahren gemäss den Ansprüchen 1-4, wobei A eine Carboxylgruppe oder ein reaktionsfähiges Derivat davon und B eine Aminogruppe bedeutet.

**6.** Verfahren gemäss Anspruch 5 zur Herstellung von 5,6,7,8-Tetrahydro-5,5,8,8-tetramethyl-4'-(2-morpholinoäthoxy)-2-naphthalin-carboxanilid.

**7.** Verfahren gemäss Anspruch 5 zur Herstellung von 5,6,7,8-Tetrahydro-5,5,8,8-tetramethyl-4'-hydroxy-2-naphthalincarboxanilid.

**8.** Verfahren gemäss Anspruch 5 zur Herstellung der Verbindungen 4'-Fluor-5,6,7,8-tetra-hydro-5,5,8,8-tetramethyl-2-naphthalin-carboxanilid; 4'-Fluor-1,1,3,3-tetramethyl-5-indancarboxanilid; 1,1,3,3-Tetramethyl-4'-(2-morpholinoäthoxy) -5-indancarboxanilid; 4'-Hydroxy-1,1,3,3-tetramethyl-5-indancarboxanilid; 6,7,8,9-Tetrahydro-7,7-dimethyl-5H-benzocyclohepten-2-carbonsäure(4-hydroxy)anilid; 6,7,8,9-Tetrahydro-7,7-dimethyl-5H-benzocyclohepten -2-carbonsäure(4-fluor)anilid;

**9.** Verfahren gemäss den Ansprüchen 1-4, wobei A eine Aminogruppe und B eine Carboxylgruppe oder ein reaktionsfähiges Derivat davon bedeutet.

**10.** Verfahren gemäss Anspruch 9 zur Herstellung von (5,6,7,8-Tetrahydro-5,5,8,8-tetramethyl-2-naphthyl)-4-hydroxybenzamid.

**11.** Verfahren gemäss Anspruch 9 zur Herstellung der Verbindungen p-Fluor-N-(5,6,7,8-tetrahydro-5,5,8,8-tetramethyl -2-naphthyl)benzamid; p-(2-Morpholinomethoxy)-N-(5,6,7,8-tetrahydro-5,5,8,8-tetramethyl-2-naphthyl)benzamid.

**12.** Verfahren gemäss Anspruch 1 zur Herstellung der Verbindungen p-Fluor-N-(1,1,3,3-tetramethyl -5-indanyl)benzamid; p-(2-Morpholinoäthoxy)-N-(1,1,3,3-tetramethyl -5-indanyl)benzamid; p-Hydroxy-N-(1,1,3, 3-tetramethyl -5-indanyl)benzamid; 6,7,8,9-Tetrahydro-9,9-dimethyl-5H-benzocyclohepten-2-carbonsäure(4-hydroxy)anilid; 6,7,8,9-Tetrahydro-9,9-dimethyl-5H-benzocyclohepten-2-carbonsäure(4-fluor)anilid; 3-Aethyl-6,7,8,9-tetrahydro-7,7-dimethyl-5H-benzocyclohepten -2-carbonsäure(4-hydroxy)anilid.

**13.** Verfahren zur Herstellung pharmazeutischer Präparate, dadurch gekennzeichnet, dass man eine Verbindung der Formel I mit üblichen pharmazeutischen Hilfs- und Trägerstoffen vermischt und in eine zur therapeutischen Anwendung geeignete Form bringt.

**14.** Verwendung einer Verbindung der Formel I zur Herstellung von pharmazeutischen Präparaten zur Behandlung von Neoplasien und Dermatosen.

## Claims

### Claims for the following Contracting States: AT, BE, CH, DE, DK, FR, GB, IT, LI, LU, NL, SE

1. Compounds of the general formula

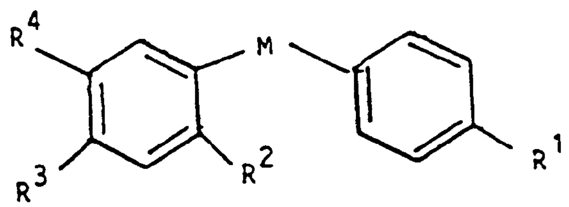

I

wherein $R^1$ represents halogen or $OR^5$; $R^2$ represents hydrogen, lower-alkyl, lower-alkoxy or halogen; $R^3$ and $R^4$ each independently represent lower-alkyl; or taken together represent alkylene with 3-5 C atoms in a straight-chain; $R^5$ signifies hydrogen, acyl, lower-alkoxycarbonyl or lower-alkyl, which can be substituted by amino, mono- or di-alkylamino or a residue -N-Het; -N-Het signifies a 5-8-membered, saturated or unsaturated monocyclic heterocycle attached via a N atom; and M signifies -CONH- or -NHCO-.

2. Compounds in accordance with claim 1, wherein $R^3$ and $R^4$ taken together represent alkylene with 3-5 C atoms in a straight chain.

3. Compounds in accordance with claim 2, wherein $R^3$ and $R^4$ taken together are the residues $-C(CH_3)_2-CH_2-C(CH_3)_2-$, $-C(CH_3)_2-CH_2CH_2-C(CH_3)_2-$ and $-CH_2CH_2-C(CH_3)_2-CH_2CH_2-$.

4. Compounds in accordance with claims 1-3, wherein $R^1$ is hydroxy, fluorine, morpholinoethoxy or N-methylpiperidinoethoxy.

5. Compounds in accordance with claims 1-4, wherein M is -CONH-.

6. 5,6,7,8-Tetrahydro-5,5,8,8-tetramethyl-4'-(2-morpholinoethoxy)-2-naphthalenecarboxanilide.

7. 5,6,7,8-Tetrahydro-5,5,8,8-tetramethyl-4'-hydroxy-2-naphthalenecarboxanilide.

8. The compounds 4'-fluoro-5,6,7,8-tetrahydro-5,5,8,8-tetramethyl-2-naphthalenecarboxanilide; 4-fluoro-1,1,3,3-tetramethyl-5-indanecarboxanilide; 1,1,3,3-tetramethyl-4'-(2-morpholinoethoxy)-5-indanecarboxanilide; 4'-hydroxy-1,1,3,3-tetramethyl-5-indanecarboxanilide; 6,7,8,9-tetrahydro-7,7-dimethyl-5H-benzocycloheptene-2-carboxylic acid (4-hydroxy)anilide; 6,7,8,9-tetrahydro-7,7-dimethyl-5H-benzocycloheptene-2-carboxylic acid (4-fluoro)anilide.

9. Compounds in accordance with claims 1-4, wherein M is -NHCO-.

10. (5,6,7,8-Tetrahydro-5,5,8,8-tetramethyl-2-naphthyl)-4-hydroxybenzamide.

11. The compounds p-fluoro-N-(5,6,7,8-tetrahydro-5,5,8,8-tetramethyl-2-naphthyl)benzamide; p-(2-morpholinomethoxy)-N-(5,6,7,8-tetrahydro-5,5,8,8-tetramethyl-2-naphthyl)benzamide.

12. The compounds p-fluoro-N-(1,1,3,3-tetramethyl-5-indanyl)benzamide; p-(2-morpholinoethoxy)-N-(1,1,3,3-tetramethyl-5-indanyl)benzamide; p-hydroxy-N-(1,1,3,3-tetramethyl-5-indanyl)benzamide; 6,7,8,9-tetrahydro-9,9-dimethyl-5H-benzocycloheptene-2-carboxylic acid (4-hydroxy)anilide; 6,7,8,9-tetrahydro-9,9-dimethyl-5H-benzocycloheptene-2-carboxylic acid (4-fluoro)anilide; 3-ethyl-6,7,8,9-tetrahydro-7,7-dimethyl-5H-benzocyclohepten-2-carboxylic acid (4-hydroxy)anilide.

13. The compounds of formula I for use as medicaments.

14. A process for the manufacture of the compounds of claim 1, characterized by
a) reacting a compound of the general formula

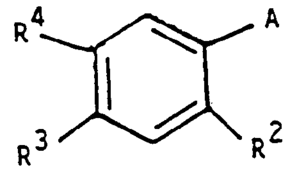

II

with a compound of the general formula

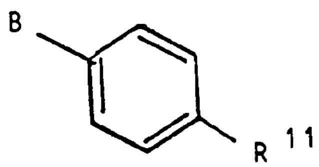

III

wherein either A signifies a carboxyl group or a reactive derivative thereof and B signifies an amino group or A signifies an amino group and B signifies a carboxyl group or a reactive derivative thereof and $R^{11}$ signifies a residue $R^1$ in which an amino group which may be present is in protected form, whereupon an amino protecting group which may be present is cleaved off.

**15.** Pharmaceutical preparations containing a compound of formula I and usual carrier materials and adjuvants.

**16.** The use of a compound of formula I for the manufacture of pharmaceutical preparations for the treatment of neoplasms and dermatoses.

**Claims for the following Contracting States: ES, GR**

**1.** A process for the manufacture of compounds of the general formula

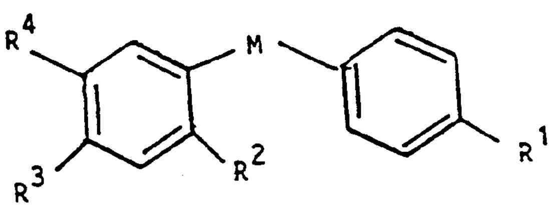

I

wherein $R^1$ represents halogen or $OR^5$ ; $R^2$ represents hydrogen, lower-alkyl, lower-alkoxy or halogen; $R^3$ and $R^4$ each independently represent lower-alkyl or taken together represent alkylene with 3-5 C atoms in a straight-chain; $R^5$ signifies hydrogen, acyl, lower-alkoxycarbonyl or lower-alkyl, which can be substituted by amino, mono- or di-alkylamino or a residue -N-Het; -N-Het signifies a 5-8-membered, saturated or unsaturated monocyclic heterocycle attached via a N atom; and M signifies -CONH- or -NHCO-,
characterized by
a) reacting a compound of the general formula

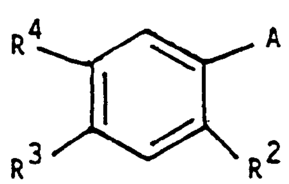

II

with a compound of the general formula

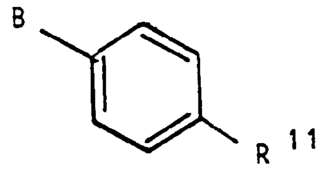

III

wherein either A signifies a carboxyl group or a reactive derivative thereof and B signifies an amino group or A signifies an amino group and B signifies a carboxyl group or a reactive derivative thereof and $R^{11}$ signifies a residue $R^1$ in which an amino group which may be present is in protected form, whereupon an amino protecting group which may be present is cleaved off.

**2.** A process in accordance with claim 1, wherein $R^3$ and $R^4$ taken together represent alkylene with 3-5 C atoms in a straight chain.

**3.** A process in accordance with claim 2, wherein $R^3$ and $R^4$ taken together are the residues $-C(CH_3)_2-CH_2-C(CH_3)_2-$, $-C(CH_3)_2-CH_2CH_2-C(CH_3)_2-$ and $-CH_2CH_2-C(CH_3)_2-CH_2CH_2-$.

**4.** A process in accordance with claims 1-3, wherein $R^1$ is hydroxy, fluorine, morpholinoethoxy or N-methylpiperidinoethoxy.

**5.** A process in accordance with claims 1-4, wherein A signifies a carboxyl group or a reactive derivative thereof and B signifies an amino group.

**6.** A process in accordance with claim 5 for the manufacture of 5,6,7,8-tetrahydro-5,5,8,8-tetramethyl-4'-(2-morpholinoethoxy)-2-naphthalenecarboxanilide.

**7.** A process in accordance with claim 5 for the manufacture of 5,6,7,8-tetrahydro-5,5,8,8-tetramethyl-4'-hydroxy-2-naphthalenecarboxanilide.

**8.** A process in accordance with claim 5 for the manufacture of the compounds 4'-fluoro-5,6,7,8-tetrahydro-5,5,8,8-tetramethyl-2-naphthalenecarboxanilide; 4'-fluoro-1,1,3, 3-tetramethyl-5-indanecarboxanilide; 1,1,3,3-tetramethyl-4'-(2-morpholinoethoxy)-5-indanecarboxanilide; 4'-hydroxy-1,1,3,3-tetramethyl-5-indanecarboxanilide; 6,7,8,9-tetrahydro-7,7-dimethyl-5H-benzocycloheptene-2-carboxylic acid (4-hydroxy)anilide; 6,7,8,9-tetrahydro-7,7-dimethyl-5H-benzocycloheptene-2-carboxylic acid (4-fluoro)anilide.

**9.** A process in accordance with claims 1-4, wherein A signifies an amino group and B signifies a carboxyl group or a reactive derivative thereof.

**10.** A process in accordance with claim 9 for the manufacture of (5,6,7,8-tetrahydro-5,5,8,8-tetramethyl-2-naphthyl)-4-hydroxybenzamide.

**11.** A process in accordance with claim 9 for the manufacture of the compounds p-fluoro-N-(5,6,7,8-tetrahydro-5,5,8,8-tetramethyl-2-naphthyl)benzamide; p-(2-morpholinomethoxy)-N-(5,6,7,8-tetrahydro-5,5,8,8-tetramethyl-2-naphthyl)benzamide.

**12.** A process in accordance with claim 1 for the manufacture of the compounds p-fluoro-N-(1,1,3,3-tetramethyl-5-indanyl)benzamide; p-(2-morpholinoethoxy)-N-(1,1,3,3-tetramethyl-5-indanyl)benzamide; p-hydroxy-N-(1,1,3,3-tetramethyl-5-indanyl)benzamide; 6,7,8,9-tetrahydro-9,9-dimethyl-5H-benzocycloheptene-2-carboxylic acid (4-hydroxy)anilide; 6,7,8,9-tetrahydro-9,9-dimethyl-5H-benzocycloheptene-2-carboxylic acid (4-fluoro)anilide; 3-ethyl-6,7,8,9-tetrahydro-7,7-dimethyl-5H-benzocyclohepten-2-carboxylic acid (4-hydroxy)anilide.

**13.** A process for the manufacture of pharmaceutical preparations, characterized by mixing a compound of formula I with usual pharmaceutical adjuvants and carrier materials and bringing the mixture into a form suitable for therapeutic use.

**14.** The use of a compound of formula I for the manufacture of pharmaceutical preparations for the treatment of neoplasms and dermatoses.

## Revendications

**Revendications pour les Etats contractants suivants : AT, BE, CH, DE, DK, FR, GB, IT, LI, LU, NL, SE**

1. Composés de formule générale

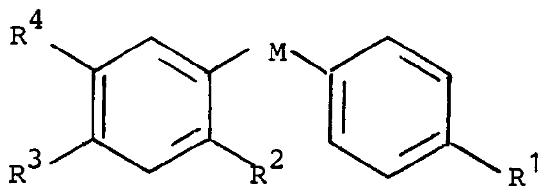

I

dans laquelle $R^1$ représente un halogène ou $OR^5$; $R^2$ représente l'hydrogène, un groupe alkyle inférieur, alcoxy inférieur ou un halogène; $R^3$ et $R^4$ représentent chacun, indépendamment l'un de l'autre, un groupe alkyle inférieur, ou forment ensemble un groupe alkylène en C 3-C 5 à chaine droite; $R^5$ représente l'hydrogène, un groupe acyle, (alcoxy inférieur)-carbonyle ou alkyle inférieur qui peut être substitué par un groupe amino, mono- ou di-alkylamino ou par un groupe -N-Het; -N-Het représente un hétérocycle monocyclique saturé ou insaturé de 5 à 8 chaînons relié par l'intermédiaire d'un atome d'azote; et M représente -CONH- ou -NHCO-.

2. Composés selon revendication 1, pour lesquels $R^3$ et $R^4$ forment ensemble un groupe alkylène à chaine droite en C 3-C 5.

3. Composés selon revendication 2, pour lesquels $R^3$ et $R^4$ forment ensemble les groupes $-C(CH_3)_2-CH_2-C(CH_3)_2-$, $-C(CH_3)_2-CH_2CH_2-C(CH_3)_2-$ et $-CH_2CH_2-C(CH_3)_2-CH_2CH_2-$.

4. Composés selon les revendications 1 à 3, pour lesquels $R^1$ représente un groupe hydroxy, le fluor, un groupe morpholinoéthoxy ou N-méthylpipéridinoéthoxy.

5. Composés selon les revendications 1 à 4, pour lesquels M représente -CONH- .

6. Le 5,6,7,8-tétrahydro-5,5,8,8-tétraméthyl-4'-(2-morpholinoéthoxy)-2-naphtalène-carboxanilide.

7. Le 5,6,7,8-tétrahydro-5,5,8,8-tétraméthyl-4'-hydroxy-2-naphtalène-carboxanilide.

8. Les composés : 4'-fluoro-5,6,7,8-tétrahydro-5,5,8,8-tétraméthyl-2-naphtalène-carboxanilide; 4'-fluoro-1,1,3,3-tétraméthyl-5-indane-carboxanilide; 1,1,3,3-tétraméthyl-4'-(2-morpholinoéthoxy)-5-indane-carboxanilide; 4'-hydroxy-1,1,3,3-tétraméthyl-5-indanecarboxanilide; 6,7,8, 9-tétrahydro-7,7-diméthyl-5H-benzocycloheptène-2-carboxy-(4-hydroxy)-anilide; 6,7,8,9-tétrahydro-7,7-diméthyl-5H-benzocycloheptène-2-carboxy-(4-fluoro)-anilide.

9. Composés selon revendications 1 à 4, pour lesquels M représente -NHCO-.

10. Le (5,6,7,8-tétrahydro-5,5,8,8-tétraméthyl-2-naphtyl)-4-hydroxybenzamide.

11. Les composés : p-fluoro-N-(5,6,7,8-tétrahydro-5,5,8,8-tétraméthyl-2-naphtyl)benzamide; p-(2-morpholino-méthoxy)-N-(5,6,7,8-tétrahydro-5,5,8,8-tétraméthyl-2-naphtyl)-benzamide.

12. Les composés : p-fluoro-N-(1,1,3,3-tétraméthyl-5-indanyl)-benzamide; p-(2-morpholinoéthoxy)-N-(1,1 , 3,3-tétraméthyl-5-indanyl)-benzamide; p-hydroxy-N-(1,1,3,3-tetraméthyl-5-indanyl)-benzamide; 6,7,8,9-tétrahydro-9,9-diméthyl-5H-benzocycloheptène-2-carboxy-(4-hydroxy)anilide; 6,7,8,9-tétrahydro-9,9-diméthyl-5H-benzocycloheptène-2-carboxy-(4-fluoro)-anilide; 3-éthyl-6,7,8,9-tétrahydro-7,7-diméthyl-5H-benzocycloheptène-2-carboxy-(4-hydroxy)-anilide.

13. Les composés de formule I, pour l'utilisation en tant que médicaments.

14. Procédé de préparation des composés de la revendication 1, caractérisé en ce que :

a) on fait réagir un composé de formule générale

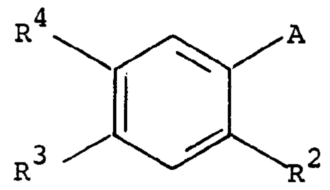

II

avec un composé de formule générale

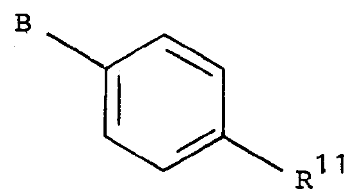

III

A représentant un groupe carboxyle ou un dérivé réactif de ce groupe et B un groupe amino, ou bien A représentant un groupe amino et B un groupe carboxyle ou un dérivé réactif de ce groupe, et $R^{11}$ représente un groupe $R^1$ dans lequel, le cas échéant, un groupe amino présent est à l'état protégé, après quoi on élimine le groupe protecteur éventuel du groupe amino.

**15.** Compositions pharmaceutiques contenant un composé de formule I et des véhicules et produits auxiliaires usuels.

**16.** Utilisation d'un composé de formule I pour la préparation de compositions pharmaceutiques prévues pour le traitement des néoplasies et des dermatoses.

**Revendications pour les Etats contractants suivants : ES, GR**

**1.** Procédé de préparation des composés de formule générale

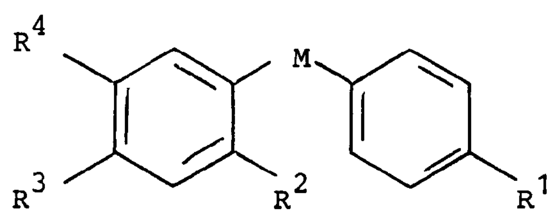

I

dans laquelle $R^1$ représente un halogène ou $OR^5$; $R^2$ représente l'hydrogène, un groupe alkyle inférieur, alcoxy inférieur ou un halogène; $R^3$ et $R^4$ représentent chacun, indépendamment l'un de l'autre, un groupe alkyle inférieur, ou forment ensemble un groupe alkylène en C 3-C 5 à chaine droite; $R^5$ représente l'hydrogène, un groupe acyle, (alcoxy inférieur)-carbonyle ou alkyle inférieur qui peut être substitué par un groupe amino, mono- ou di-alkylamino ou par un groupe -N-Het; -N-Het représente un hétérocycle monocyclique saturé ou insaturé de 5 à 8 chaînons relié par l'intermédiaire d'un atome d'azote; et M représente -CONH- ou -NHCO-,
caractérisé en ce que :
a) on fait réagir un composé de formule générale

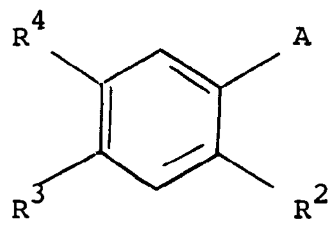

II

avec un composé de formule générale

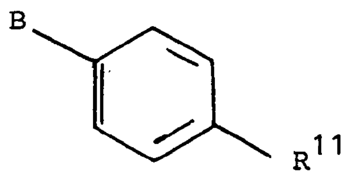

III

A représentant un groupe carboxyle ou un dérivé réactif de ce groupe et B un groupe amino, ou bien A représentant un groupe amino et B un groupe carboxyle ou un dérivé réactif de ce groupe, et $R^{11}$ représente un groupe $R^1$ dans lequel, le cas échéant, un groupe amino présent est à l'état protégé, après quoi on élimine le groupe protecteur éventuel du groupe amino.

2. Procédé selon revendication 1, dans lequel $R^3$ et $R^4$ forment ensemble un groupe alkylène à chaîne droite en C 3-C 5.

3. Procédé selon revendication 2, dans lequel $R^3$ et $R^4$ forment ensemble les groupes $-C(CH_3)_2-CH_2-C(CH_3)_2-$, $-C(CH_3)_2-CH_2CH_2-C(CH_3)_2-$ et $-CH_2CH_2-C(CH_3)_2-CH_2CH_2-$.

4. Procédé selon revendications 1 à 3, dans lequel $R^1$ représente un groupe hydroxy, le fluor, un groupe morpholinoéthoxy ou N-méthylpipéridinoéthoxy.

5. Procédé selon revendications 1 à 4, dans lequel A représente un groupe carboxyle ou un dérivé réactif de ce groupe et B un groupe amino.

6. Procédé selon revendication 5, pour la préparation du 5,6,7,8-tétrahydro-5,5,8,8-tétraméthyl-4'-(2-morpholinoéthoxy)-2-naphtalène-carboxanilide.

7. Procédé selon revendication 5, pour la préparation du 5,6,7,8-tétrahydro-5,5,8,8-tétraméthyl-4'-hydroxy-2-naphtalène-carboxanilide.

8. Procédé selon revendication 5, pour la préparation des composés : 4'-fluoro-5,6,7,8-tétrahydro-5,5,8,8-tétraméthyl-2-naphtalène-carboxanilide; 4'-fluoro-1,1,3,3-tétraméthyl-5-indane-carboxanilide; 1,1,3,3-tétraméthyl-4'-(2-morpholinoéthoxy)-5-indane-carboxanilide; 4'-hydroxy-1,1,3,3-tétraméthyl-5-indane-carboxanilide; 6,7,8,9-tétrahydro-7,7-diméthyl-5H-benzocycloheptène-2-carboxy-(4-hydroxy)-anilide; 6,7,8,9-tétrahydro-7,7-diméthyl-5H-benzocycloheptène-2-carboxy-(4-fluoro)-anilide.

9. Procédé selon les revendications 1 à 4, dans lequel A représente un groupe amino et B un groupe carboxyle ou un dérivé réactif de ce groupe.

10. Procédé selon revendication 9, pour la préparation du (5,6,7,8-tétrahydro-5,5,8,8-tétraméthyl-2-naphtyl)-4-hydroxybenzamide.

11. Procédé selon revendication 9, pour la préparation des composés : p-fluoro-N-(5,6,7,8-tétrahydro-5,5,8,8-tétraméthyl-2-naphtyl)benzamide; p-(2-morpholino-méthoxy)-N-(5,6,7,8-tétrahydro-5,5,8,8-tétraméthyl-2-naphtyl)-benzamide.

12. Procédé selon revendication 1, pour la préparation des composés : p-fluoro-N-(1,1,3,3-tétraméthyl-5-indanyl)-benzamide; p-(2-morpholinoéthoxy)-N- (1,1,3,3-tétraméthyl-5-indanyl)-benzamide; p-hydroxy-N-(1,1,3,3-tétraméthyl-5-indanyl)-benzamide; 6,7,8,9-tétrahydro-9,9-diméthyl-5H-benzocycloheptène-2-carboxy-(4-hydroxy)-anilide; 6,7,8,9-tétrahydro-9,9-diméthyl-5H-benzocycloheptène-2-carboxy-(4-fluoro)-anilide; 3-éthyl-6,7,8,9-tétrahydro-7,7-diméthyl-5H-benzocycloheptène-2-carboxy-(4-hydroxy)-anilide.

**13.** Procédé de préparation de compositions pharmaceutiques, caractérisé en ce que l'on mélange un composé de formule I avec des véhicules et produits auxiliaires pharmaceutiques usuels et on met le mélange sous une forme appropriée à l'utilisation thérapeutique.

**14.** Utilisation d'un composé de formule I pour la préparation de compositions pharmaceutiques prévues pour le traitement des néoplasies et des dermatoses.